# EUROPÄISCHE PATENTANMELDUNG

(11) **EP 1 378 244 A1**
(43) Veröffentlichungstag der Anmeldung: **07.01.2004**
(21) Anmeldenummer: 03014959.5
(22) Anmeldetag: 01.07.2003
(51) Int. Cl.: A61K 35/56, A61P 29/00, A61P 19/02

(54) **Wirkstoffmischung aus Bestandteilen der Grünlippenmuschel und zusätzlichen Omega-3-Fettsäuren**

(30) Priorität: 01.07.2002 DE 20210182 U
(71) Anmelder: Bio-Innovation Development Limited, Christchurch 1 (NZ)
(72) Erfinder: Lingens,Johann Matthias, 79-83 Hereford Street, Christchurch 1 (NZ)
(74) Vertreter: Barz, Peter, Dr.

(57) **Zusammenfassung**

Es wird eine Wirkstoffmischung beschrieben, die ein Konzentrat aus den Gonaden der Grünlippenmuschel (pema canaliculus) und eine nicht aus der Grünlippenmuschel stammende Komponente, die eine oder mehrere zusätzliche Omega-3-Fettsäuren enthält, umfasst.

## Beschreibung

Die Erfindung betrifft eine Wirkstoffmischung umfassend Bestandteile der Grünlippenmuschel (perna canaliculus), nämlich ein Konzentrat aus den Gonaden, und zusätzliche Omega-3-Fettsäuren, und ihre Verwendung.

Die Grünlippenmuschel (perna canaliculus) besitzt einen natürlichen Gehalt an Omega-3-Fettsäuren, der relativ gering ist und bei der Muschelernte Schwankungen unterliegt.

Die Grünlippenmuschel wird seit Jahrzehnten in der Behandlung von entzündlichen und degenerativen Beschwerden des Bewegungsapparats eingesetzt. Die antiinflammatorische Wirkung der Grünlippenmuschel beruht nach Erkenntnissen der Forschung u.a. auf einer modulatorischen Wirkung auf Entzündungsmediatoren in Form einer COX-2 Hemmung, wobei in der Literatur als primäre entzündungshemmende Komponente der Lipidgehalt der Grünlippenmuschel angesehen wird.

Übliche Anwendungsformen für eine entzündungshemmende Wirkung sind die Grünlippenmuschel als solche oder ein gefriergetrocknetes Pulver der ganzen Muschel. In US-A-6083536 und WO 96/05164 werden z.B. Lipidextrakte aus der Grünlippenmuschel beschrieben.

Die Aufgabe bestand in der Bereitstellung eines Präparats aus der Grünlippenmuschel, das gegenüber den bekannten Produkten ein erhöhtes Wirkpotential bezüglich einer entzündungshemmenden Wirkung aufweist.

Die Aufgabe wurde überraschenderweise gelöst durch eine Wirkstoffmischung umfassend ein Konzentrat aus den Gonaden der Grünlippenmuschel (perna canaliculus) und eine nicht aus der Grünlippenmuschel stammende Komponente, die eine oder mehrere zusätzliche Omega-3-Fettsäuren enthält.

Mit der erfindungsgemäßen Wirkstoffmischung wird überraschenderweise erreicht, das bekannte Wirkpotential der Grünlippenmuschel beim Einsatz in der Behandlung von entzündlichen und degenerativen Erscheinungen insbesondere des Bewegungsapparats (rheumatische Beschwerden) beim Menschen oder bei Tieren zu verstärken. Dadurch wird es möglich, bezogen auf eine Einheitsmenge eine höhere Wirksamkeit als bei herkömmlichen Grünlippenmuschel-Produkten zu erreichen, was eine Volumenverringerung der Mischung bei gleicher Wirkung ermöglicht.

Die Wirkstoffmischung umfasst ein Konzentrat aus den Gonaden der Grünlippenmuschel. Die Gonaden der Grünlippenmuschel werden zweckmäßigerweise aus dem frischen Muschelfleisch herausgetrennt. Unter Konzentrat wird hier ein Material verstanden, bei dem den Gonaden zumindest ein Teil der Flüssigkeit entzogen ist oder das nur bestimmte Bestandteile der Gonaden enthält (z.B. ein Extrakt). Das Konzentrat aus den Gonaden kann insbesondere auch erhalten werden, indem das Muschelfleisch aus den Schalen der frischen Muscheln gelöst wird und die Weichteile der Muschel, von denen die Gonaden ein wesentlicher Bestandteil sind, in einer wässrigen Phase aus dem Muschelfleisch herausgetrennt bzw. abgetrennt werden und anschließend die im wesentlichen Gonaden enthaltenden Weichteile (gefrier)getrocknet und gemahlt werden. Dabei werden auch die Faserbestandteile der Muscheln entfernt.

Neben dem Konzentrat enthält die Wirkstoffmischung vorzugsweise keine weiteren aus der Grünlippenmuschel stammende Komponenten, es können aber gegebenenfalls gewisse Mengen z.B. aus Produkten der ganzen Muschel zugesetzt werden. Sofern neben dem Konzentrat andere Produkte der Grünlippenmuschel enthalten sind, machen sie im allgemeinen nicht mehr als 90 Gew.-%, bevorzugter nicht mehr als 50 Gew.-% oder 20 Gew.-%, bezogen auf die Trockenmasse der in der Wirkstoffmischung enthaltenen Grünlippenmuschel-Produkte insgesamt, aus.

Das Konzentrat kann zum Beispiel aus der Trocknung der wie vorstehend beschrieben gewonnenen Gonaden erhalten werden. Besonders bevorzugt wird als Konzentrat ein Lyophilisat der Gonaden verwendet. Die Lyophilisation oder Gefriertrocknung ist ein dem Fachmann bekanntes Verfahren, bei dem ein gefriergetrocknetes Material im Vakuum durch Ausfrieren der flüchtigen Substanz (z.B. Lösungsmittel, Wasser) und dann Verdampfen im gefrorenen Zustand gewonnen wird.

Statt der Verwendung eines getrockneten oder gefriergetrockneten Produkts als Konzentrat kann auch ein Extrakt, z.B. ein Lipidextrakt, aus den Gonaden verwendet werden. Diese Extrakte werden gewöhnlich aus den getrockneten bzw. gefriergetrockneten Produkten mit einem Extraktionsmittel wie überkritischem CO₂ erhalten. Als Ausgangsprodukte können auch Homogenisate des frischen Muschelfleisches eingesetzt werden. Das Verfahren zur Herstellung dieser Extrakte ist dem Fachmann bekannt (siehe z.B. US-A-6083536 oder WO 96/05164). Natürlich können auch Mischungen der beschriebenen Konzentrate verwendet werden.

Die Wirkstoffmischung umfasst ferner eine zusätzliche, Omega-3-Fettsäuren enthaltende Komponente. Omega-3-Fettsäuren sind allgemein bekannt. Beispiele sind alpha-Linolensäure (18:3), Docosapentaensäure (DPA, 22:5) und insbesondere Eicosapentaensäure (EPA, 20:5) und Docosahexaensäure (DHA, 22:6). Die Komponente kann eine oder mehrere Omega-3-Fettsäuren enthalten. Die Komponente stammt nicht von der Grünlippenmuschel. Sie dient der Anreicherung des Konzentrats mit Omega-3-Fettsäuren. Da die Komponente vorzugsweise natürlichen Ursprungs ist, kann sie neben den Omega-3-Fettsäuren auch andere Bestandteile enthalten. Natürlich kann die Komponente aber auch nur aus einer oder mehreren Omega-3-Fettsäuren bestehen.

Die Komponente ist bevorzugt pflanzlichen Ursprungs und besonders bevorzugt maritimen Ursprungs. Beispiele für Komponenten pflanzlichen Ursprungs, die Omega-3-Fettsäuren enthalten, sind pflanzliche Öle wie Leinöl, Hanföl, Sojaöl, Flachsöl, Nachtkerzenöl, Rapsöl und Walnussöl oder Produkte (z.B. Extrakte) daraus. Fische und Meeresfrüchte oder allgemein maritime Lebewesen enthalten ebenfalls Omega-3-Fettsäuren, so dass daraus gewonnene Produkte ebenfalls verwendet werden können. Diese Komponenten maritimen Ursprungs sind bevorzugt. Es können auch Mischungen verschiedener, Omega-3-Fettsäuren enthaltener Komponenten verwendet werden, z.B. Mischungen aus Komponenten pflanzlichen und maritimen Ursprungs.

Die die zusätzlichen Omega-3-Fettsäuren enthaltende Komponente kann in fester bzw. trockener oder fluider Form vorliegen, z.B. als Öl oder Pulver. Nach einem in der Technik bekannten Verfahren kann es sich bei der Komponente um mikroverkapselte Produkte handeln. Unter Mikroverkapselung versteht man im allgemeinen z.B. eine Ummantelung fester oder flüssiger Partikel. Das Beschichtungsmaterial kann z.B. aus natürlichen (z.B. Stärke) oder synthetischen Substanzen (z.B. modifizierter Stärke) und weiteren Additiven bestehen. Solche Produkte sind im Handel erhältlich.

Bevorzugt wird Fischöl oder ein Fischöl-Pulver, das durch Mikroverkapselung erhalten wird, als Omega-3-Fettsäuren enthaltende Komponente eingesetzt. Durch die Mikroverkapselung wird das Fischöl z.B. vor Oxidation geschützt. Natürlich umfasst die Mischung dann auch die in der Ummantelung enthaltenen Stoffe.

Das Verhältnis von Konzentrat aus den Gonaden der Grünlippenmuschel zu der zusätzlichen Komponente, die eine oder mehre Omega-3-Fettsäuren enthält, kann in weiten Bereichen variiert werden. Im allgemeinen wird ein solches Mischungsverhältnis gewählt, dass das Gewichtsverhältnis von Konzentrat aus den Gonaden (Trockengewicht) zu den Omega-3-Fettsäuren, die in der nicht aus der Grünlippenmuschel stammenden Komponente enthalten sind, z.B. im Bereich von 100:1 bis 1:30 liegt. Ein bevorzugter Bereich für das Gewichtsverhältnis ist 3:1 bis 1:3.

Die Wirkstoffmischung kann weitere übliche Komponenten enthalten, z.B.
Mineralstoffe, Vitamine, Spurenelemente, Nährstoffe, Antioxidationsmittel, Stabilisatoren und/oder je nach Darreichungsform übliche Hilfsstoffe. Gegebenfalls kann die Wirkstoffmischung auch in einem Lösungsmittel, wie Wasser, einer isotonen Kochsalzlösung oder Ethanol, aufgenommen werden, um Lösungen, Emulsionen oder Suspensionen zu erhalten.

Die Wirkstoffmischung kann eine Trockenmischung, eine flüssige Mischung oder eine Emulsion oder Suspension darstellen. Bevorzugt liegt die Mischung als Paste, Fluid, Trockenmischung bzw. Pulver vor. Bevorzugte Darreichungsformen sind Kapseln, z.B. Weichgelatine- oder Hartgelatinekapseln, Dragees und bei Veterinärtherapeutika oder beim diätetischen Einsatz auch als Pulver oder in flüssiger Form. Eine weitere vorteilhafte Anwendung ist z.B. in Form von Gels, Pasten, Salben und Cremes für die lokale Applikation, z.B. zum Einreiben.

### Ausführungsbeispiel

Zur Herstellung einer Wirkstoffmischung wird ein Lyophilisat aus den Gonaden der Grünlippenmuschel mit zusätzlichen Omega-3-Fettsäuren angereichert. Hierfür wird es mit einem handelsüblichen Fischöl-Pulver gemischt. Bei dem Fischöl-Pulver handelt es sich um ein Fischöl, das reich an EPA und DHA ist (EPA/DHA = 18/12) und durch Mikroverkapselung in ein Pulver überführt wurde.

## Patentansprüche

1. Wirkstoffmischung umfassend ein Konzentrat aus den Gonaden der Grünlippenmuschel (perna canaliculus) und eine nicht aus der Grünlippenmuschel stammende Komponente, die eine oder mehrere zusätzliche Omega-3-Fettsäuren enthält.

2. Wirkstoffmischung nach Anspruch 1, **dadurch gekennzeichnet, dass** das Konzentrat ein Lyophilisat oder ein Extrakt aus den Gonaden der Grünlippenmuschel ist.

3. Wirkstoffmischung nach einem der Ansprüche 1 und 2, **dadurch gekennzeichnet, dass** die die zusätzlichen Omega-3-Fettsäuren enthaltende Komponente maritimen Ursprungs ist.

4. Wirkstoffmischung nach Anspruch 3, **dadurch gekennzeichnet, dass** die die zusätzlichen Omega-3-Fettsäuren enthaltende Komponente Fischöl oder Fischöl-Pulver ist.

5. Wirkstoffmischung nach einem der Ansprüche 1 und 2, **dadurch gekennzeichnet, dass** die die zusätzlichen Omega-3-Fettsäuren enthaltende Komponente pflanzlichen Ursprungs ist.

6. Wirkstoffmischung nach einem der Ansprüche 1 bis 5, **dadurch gekennzeichnet, dass** die die zusätzlichen Omega-3-Fettsäuren enthaltende Komponente maritimen und pflanzlichen Ursprungs ist.

7. Wirkstoffmischung nach einem der Ansprüche 1 bis 6, **dadurch gekennzeichnet, dass** die die zusätzlichen Omega-3-Fettsäuren enthaltende Komponente fest oder fluid ist.

8. Wirkstoffmischung nach Anspruch 7, **dadurch gekennzeichnet, dass** die die zusätzlichen Omega-3-Fettsäuren enthaltende Komponente ein durch Mikroverkapselung erhaltenes Pulver ist.

9. Wirkstoffmischung nach einem der Ansprüche 1 bis 8, **dadurch gekennzeichnet, dass** sie ein Fluid, eine Paste, ein Pulver oder eine Trockenmischung ist.

10. Wirkstoffmischung nach einem der Ansprüche 1 bis 9, **dadurch gekennzeichnet, dass** sie ferner Mineralstoffe, Vitamine, Spurenelemente, Nährstoffe, Antioxidationsmittel, Stabilisatoren und/oder je nach Darreichungsform übliche Hilfsstoffe umfasst.

11. Verwendung der Wirkstoffmischung nach einem der Ansprüche 1 bis 10 zur Behandlung von entzündlichen und degenerativen Krankheitszuständen.
